# EUROPÄISCHE PATENTSCHRIFT

(11) **EP 2 560 506 B1**
(45) Veröffentlichungstag und Bekanntmachung des Hinweises auf die Patenterteilung: **08.10.2014**
(21) Anmeldenummer: 10715534.3
(22) Anmeldetag: 21.04.2010
(51) Int. Cl.: C12P 39/00, A23L 1/218, A23L 1/23, A23L 1/36, A23L 2/38, A23L 1/20

(54) **VERFAHREN ZUR HERSTELLUNG EINES FERMENTIERTEN NATURPRODUKTS**
PROCESS FOR PRODUCING A FERMENTED NATURAL PRODUCT
PROCÉDÉ DE PRODUCTION D'UN PRODUIT NATUREL FERMENTÉ

(43) Veröffentlichungstag der Anmeldung: 27.02.2013
(73) Patentinhaber: Niedermaier-May, Cordula, 85662 Hohenbrunn (DE)
(72) Erfinder: Niedermaier-May, Cordula, 85662 Hohenbrunn (DE)
(74) Vertreter: Dennemeyer & Associates S.A.
(86) Internationale Anmeldenummer: PCT/EP2010/055267
(87) Internationale Veröffentlichungsnummer: WO 2011/131238

(56) Entgegenhaltungen:
- EP-A2- 1 153 549
- WO-A1-2007/136253
- US-A- 4 385 118
- US-A1- 2010 040 732
- DATABASE WPI Week 200682 Thomson Scientific, London, GB; AN 2006-807698 XP002593172 & KR 2005 103 261 A (CHOI J H) 27. Oktober 2005 (2005-10-27)

## Beschreibung

### Gebiet der Erfindung

Die vorliegende Erfindung betrifft ein Verfahren zur Herstellung eines fermentierten Naturprodukts, insbesondere die Herstellung eines hoch wirksamen fermentierten Naturprodukts, das lange haltbar ist.

### Hintergrund der Erfindung

Als Naturprodukte werden Produkte bezeichnet, die Produkte aus der Natur darstellen, beispielsweise die Milch von Tieren. Naturprodukte sind allerdings auch solche Produkte, die aus natürlich vorkommenden Rohmaterialien, wie beispielsweise Früchten und Gemüse, hergestellt werden.

KR 2005 103 261 A beschreibt die Herstellung eines fermentierten Getränks auf Knoblauchbasis, dessen beeinträchtigender Geruch und Toxizität durch dreimalige natürliche Fermentation beseitigt werden.

US 4,385.118 beschreibt ein Verfahren zur kontinuierlichen Fermentation von Zucker zu Alkohol in einer Reihe von Fermentationsgefäßen, wobei eine Rückführung von Hefe unabhangig von den zu einem bestimmten Zeitpunkt in jedem Gefäß auftretenden Fermentationsbedinaunaen erfolgt.

In den letzten Jahren geht der Trend vermehrt zu einer besonders gesunden, ausgewogenen Ernährung. Dabei spielt der Konsum von natürlichen Produkten eine immer größer werdende Rolle.

Es ist bekannt, solche Naturprodukte herzustellen, indem man ein natürlich vorkommendes Rohmaterial oder ein Gemisch aus verschiedenen Rohmaterialien in Gegenwart von Mikroorganismen fermentiert. Ein solcher Fermentauszug wird dann gereinigt, beispielsweise durch Zentrifugation und/oder Filtration, um die unlöslichen Bestandteile zu entfernen. Gegebenenfalls wird der Fermentauszug pasteurisiert, um die Mikroorganismen zu inaktivieren.

Ein derartiges Verfahren ist aus der EP 1 153 549 B1 bekannt. Bei diesem Verfahren wird ein Gemisch aus Früchten, Gemüsen und Hülsenfrüchten als Rohmaterialien in Gegenwart von Mikroorganismen zur Herstellung eines Fermentauszugs fermentiert, wonach ein Teil des Fermentauszugs mindestens einer weiteren Fermentation unterzogen wird und dieser Teil mit dem verbleibendenden Teil des ersten Fermentauszugs vermischt wird. Nach dem Vermischen wird das Produkt auf etwa 80°C erhitzt und in geeignete Behälter abgefüllt, die dann in üblicher Weise luftdicht verschlossen werden.

Es hat sich herausgestellt, dass das nach der EP 1 153 549 B1 hergestellte Naturprodukt ein hohes antioxidatives Potenzial in Form von Polyphenolen aufweist und daher vor freien Sauerstoffradikalen im Körper schützt. Des Weiteren ist festgestellt worden, dass das Produkt die Immunabwehr im Körper durch Enzymaktivierung stärkt.

Wissenschaftliche Studien haben gezeigt, dass das nach der EP 1 153 549B1 hergestellte Naturprodukt hinsichtlich der Immunstimulation eine Sättigungs- bzw. Abklingkinetik eintritt. Dieser Aktivitätsverlust im höheren Konzentrationsbereich kann dadurch kompensiert werden, indem lebende probiotische Mikroorganismen dem Naturprodukt hinzugefügt werden. Es hat sich allerdings herausgestellt, dass die probiotischen Mikroorganismen innerhalb kürzester Zeit, das heißt innerhalb weniger

Stunden, inaktiviert werden, was auf das saure Milieu des Naturprodukts zurückzuführen ist.

Es ist daher Aufgabe der vorliegenden Erfindung, ein Verfahren zur Herstellung eines fermentierten Naturprodukts zur Verfügung zu stellen, das auch bei hohen Konzentrationen über einen langen Zeitraum hoch wirksam ist und ohne Weiteres über einen langen Zeitraum gelagert werden kann, ohne dass die enzymregelnde Aktivität des Naturprodukts abgeschwächt wird.

### Beschreibung der Erfindung

Die Aufgabe der vorliegenden Erfindung ist mit einem Verfahren zur Herstellung eines fermentierten Produkts gemäß Anspruch 1 gelöst worden.

Die vorliegende Erfindung betrifft ein Verfahren zur Herstellung eines fermentierten Naturprodukts, das die Schritte aufweist:
a) Fermentieren von Rohmaterialien für das Naturprodukt, die aus Früchten, Gemüsen, Hülsenfrüchten, Pilzen, Nüssen, Getreiden, Reis, Kräutern, Wurzeln, Blättern, Blüten, einzeln oder in Kombination gewählt sind, in Gegenwart von Mikroorganismen in einem Hauptfermenter bei einer Temperatur von 20 bis 35°C zur Herstellung eines ersten Fermentauszugs;
b) Entnehmen mindestens eines Teils des ersten Fermentauszugs und Überführen dieses Teils in mindestens einen weiteren Nebenfermenter, wobei der entnommene Teil bis zu einem Drittel des ersten Fermentauszugs bildet;
c) Fermentieren dieses Teils des Fermentauszugs in Gegenwart von Mikroorganismen bei einer Temperatur von 20 bis 35°C zur Bildung von mindestens einem Teilfermentauszug;
d) Überführen dieses mindestens einen Teilfermentauszugs in den Hauptfermenter und Vermischen mit dem restlichen ersten Fermentauszug und
e) Zugeben eines Propagats aus Mikroorganismen in die vereinten Fermentauszüge, wobei in den Schritten a) und c) die Mikroorganismen in einer Menge von 10⁶ bis 10¹² Zellen/ml, bevorzugt 10⁸ bis 10¹⁰ Zellen/ml, hinzugefügt werden.

Die Unteransprüche betreffen bevorzugte Ausführungsformen des erfindungsgemäßen Verfahrens.

Mit dem erfindungsgemäßen Verfahren ist es gelungen, ein konzentriertes, fermentiertes Naturprodukt zur Verfügung zu stellen, das die Enzyme der Immunabwehr des Körpers signifikant aktiviert. Diese Eigenschaft bleibt auch nach der Haltbarmachung durch eine Kurzzeit-Hitzebehandlung erhalten. Das abgefüllte fermentierte Naturprodukt lässt sich ohne Weiteres für einen Zeitraum von mindestens drei Jahren lagern, ohne dass das Naturprodukt an Aktivität verliert.

### Kurze Beschreibung der Zeichnungen

Die vorliegende Erfindung wird mit Bezugnahme auf die Zeichnungen näher erläutert. Es zeigen
Figur 1 ein Prozessschema, das eine Ausführungsform des erfindungsgemäßen Verfahrens gemäß Variante A erläutert;
Figur 2 ein Prozessschema für eine Ausführungsform der Prozessvariante B des erfindungsgemäßen Verfahrens;
Figur 3 ein Diagramm, das die immunmodulierende Aktivität eines Naturprodukts des Standes der Technik beschreibt und
Figur 4 ein Diagramm, das die immunmodulierende Aktivität von erfindungsgemäß hergestellten Produkten beschreibt;
Figur 5 ein Diagramm, das die immunmodulierende Aktivität der erfindungsgemäß hergestellten Produkte mit einem Naturprodukt des Standes der Technik zeigt.

### Detaillierte Beschreibung der Erfindung

Das erfindungsgemäße Verfahren stellt ein fermentiertes Naturprodukt zur Verfügung, das aufgrund der Anreicherung mit Mikroorganismen in den vereinten Fermentauszügen seine immunmodulierenden Aktivitäten über einen langen Zeitraum erhält. Auch nach der Hitzeinaktivierung, d. h. im hitzeinaktiviertem Zustand, bewirken die hinzugefügten Mikroorganismen mit den ebenfalls hitzeinaktivierten Mikroorganismen aus den Fermentationen additive Effekte der Aktivierung. Diese Effekte wurden experimentell wie folgt nachgewiesen.

Zur Durchführung der Tests zur Erfassung von immunmodulierenden Aktivitäten wurde das ACC/Vollblut-Testsystem angewendet. Mit diesem Testsystem wird eine Entzündungsreaktion durch Aktivierung von neutrophilen Granulocyten im Vollblut simuliert.

Der Test basiert darauf, dass in frischem Humanblut in Gegenwart von Immunaktivatoren die Bildung von aktivierten neutrophilen Granulocyten nachgewiesen werden kann. Die im Blut befindlichen Granulocyten erkennen die Immunaktivatoren und werden in einen aktivierten Zustand überführt. Im Aktivierungszustand produzieren die neutrophilen Granulocyten aktive Sauerstoffspezies, wobei Wasserstoffperoxid mit Chlorid durch das Enzym Myeloperoxidase (MPO) zur unterchloriger Säure (HOCl) umgesetzt wird. Die unterchlorige Säure wird dann mit 1-Aminocyclopropan-1-caboxylsäure (ACC) als Indikatormolekül zu Ethen und anderen Produkten umgesetzt. Das gebildete Ethen ist ein Maß für die ausgelöste Entzündungsreaktion und wird gaschromatisch nachgewiesen.

Im biochemischen Modell wird das Vollblut mit den zu testenden Substanzen für 30 Minuten mit dem Indikatormolekül ACC in volumengeeichten, gasdicht verschlossenen Reaktionsgefäßen inkubiert. Anschließend wird das durch ACC freigesetzte Ethen gaschromatografisch quantifiziert.

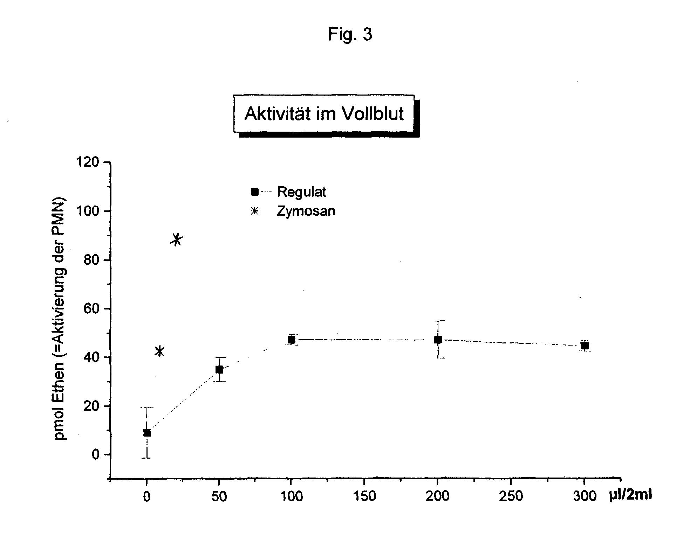

Figur 3 zeigt ein Diagramm zur Aktivierung von neutrophilen Granulocyten im Vollblut. Gemessen wurde die immunmodulierende Aktivität von Regulat, einem nach der EP 1 153 549 B1 hergestellten Naturprodukt. Als Referenz wurde zusätzlich eine Probe mit Zymosan (ein Zellwandpräparat aus Hefe), das ein experimenteller Aktivator der angeborenen Immunantwort (innate immunity) ist, getestet. Die Konzentration an hitzeaktivierten, toten Mikroorganismen liegt in einem Bereich von 0 bis 300 µl/2 ml Vollblut. Dieses entspricht einer Menge von 5 x10¹⁰ bis 2,5 x10¹¹ Zellen. Das Zymosan wurde als Zymosan 50 mit 200 µg/2 ml Vollblut getestet.

Wie aus Figur 3 zu entnehmen ist, erfolgt beim Regulat eine Aktivierung der Immunreaktion, die sprunghaft bis 100 µl/2 ml Vollblut ansteigt, wonach dann bei höheren Konzentrationen eine Sättigung bzw. ein Abklingen der Immunreaktion eintritt. Bei Zymosan tritt erwartungsgemäß eine sprunghafte Aktivierung der Immunantwort bereits bei geringsten Mengen ein. Die Ethenbindung wird in pmol/60 Minuten gemessen.

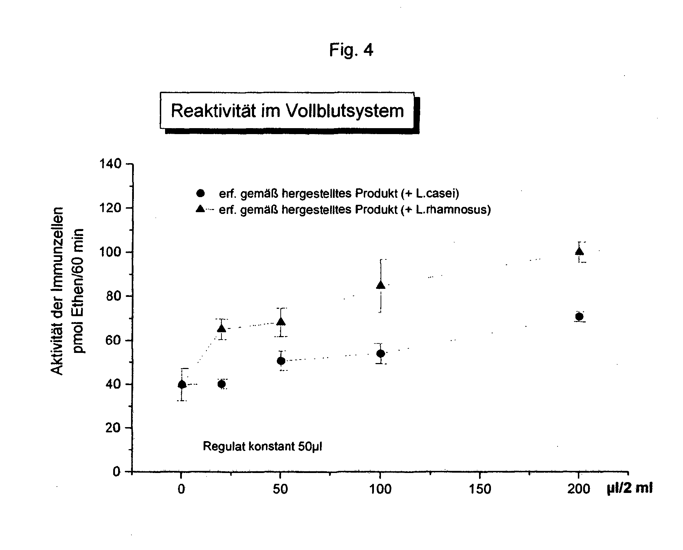

Das Diagramm von Figur 4 zeigt die immunstimmulierende Reaktivität von zwei erfindungsgemäß hergestellten Produkten im Vollblutsystem. Die Punkt-gestrichelte Kurve zeigt ein erfindungsgemäß hergestelltes Produkt, bei dem nach der Fermentierung in die vereinten Fermentauszüge ein Propagat aus Lactobacillus casei hinzugefügt wurde. Die Dreieck-gestrichelte Kurve zeigt ein weiteres erfindungsgemäß hergestelltes Produkt, bei dem ein Propagat aus Lactobacillus rhamnosus nach der Fermentation hinzugefügt wurde. Es ist deutlich am Anstieg der Ethenbildung zu erkennen, dass bei zunehmender Konzentration additive Effekte der Immunaktivierung bewirkt werden. Die Sättigungs- bzw. Abklingkinetik, die in Figur 3 mit dem Produkt des Standes der Technik festgestellt wurde, ist völlig beseitigt. Insbesondere bei 100 bis 200 µl/2 ml Vollblut wird ein signifikanter Anstieg der Immunantwort festgestellt. Es ist also eine kontinuierliche Aktivitätssteigerung zu verzeichnen, die beide Mikroorganismen allein nicht zeigen.

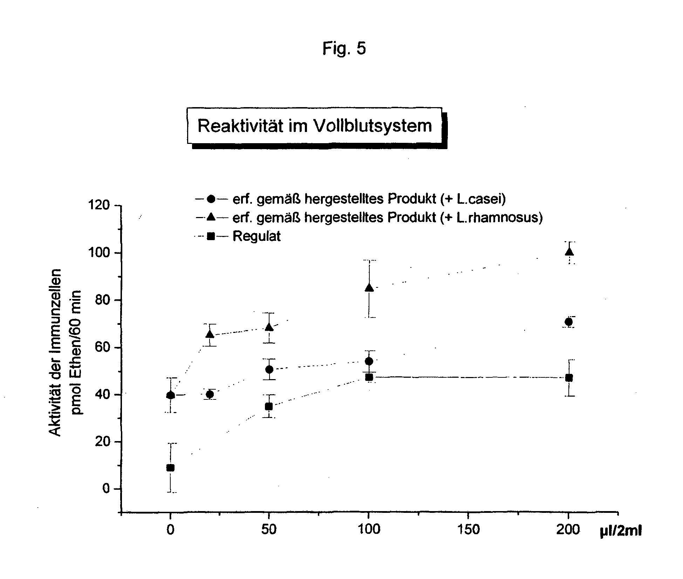

Figur 5 zeigt die Reaktivitäten im Vollblutsystem in direktem Vergleich. Wie deutlich zu erkennen ist, erreichen die erfindungsgemäß hergestellten Naturprodukte über einen Konzentrationsbereich von 0 bis 200µl/2ml Aktivitätssteigerungen, während das Produkt des Standes der Technik nach 100µl/2ml in seiner immunmodulierenden Aktivität stagniert und sogar abfällt. Es ist festgestellt worden, dass die Zugabe von Lactobacillus rhamnosus im Propagat die immunstimulierende Wirkung stärker beeinflusst als Lactobacillus casei.

Nach der Zugabe des Propagats aus Mikroorganismen in die vereinten Fermentauszüge können die Mikroorganismen enthaltenden Fermentauszüge bei einer Temperatur im Bereich von 65°C bis 75°C hitzebehandelt werden, um die darin enthaltenden Mikroorganismen zu inaktivieren. Das nach dem erfindungsgemäßen Verfahren hergestellte Naturprodukt enthält somit keine Mikroorganismen, die sich noch vermehren können. In einer besonderen Ausführungsform der vorliegenden Erfindung wird die Hitzebehandlung bei einer Temperatur im Bereich von 68°C bis 72°C durchgeführt. Diese Hitzebehandlung ist eine Kurzzeiterhitzung, die bevorzugt in einem Zeitraum von 60 bis 360 Sekunden, vorzugsweise 120 bis 300 Sekunden durchgeführt wird.

Nach der Zugabe des Propagats und ggf. nach der Kurzzeiterhitzung wird das Endprodukt in eine Füllvorrichtung geleitet, wo in geeignete Behältnisse, wie beispielsweise Flaschen, abgefüllt werden. Die Flaschen werden dann in üblicher Weise luftdicht verschlossen.

Die vereinten Fermentauszüge mit den darin enthaltenen Mikroorganismen weisen einen pH-Wert von 3,2 bis 4 auf.

In dem erfindungsgemäßen Verfahrens werden in den Schritten a) und c) die Mikroorganismen in einer Menge von 10⁶ bis 10¹² Zellen/ml, bevorzugt 10⁸ bis 10¹⁰ Zellen/ml hinzugefügt.

Es hat sich als vorteilhaft für das erfindungsgemäße Verfahren erwiesen, wenn das Propagat aus Mikroorganismen in der Stufe e) in einer Menge in die vereinten Fermentauszüge hinzugefügt wird, die etwa zur Verdopplung der Mikroorganismenzahl führt.

Das Propagat aus Mikroorganismen wird in einem Propagator angezüchtet. In der Regel werden zunächst die Mikroorganismen mit einer Starterkultur in dem Propagator kultiviert und auf eine Konzentration von 10¹² bis 10¹⁶ cfu/ml, bevorzugt 10¹³ bis 10¹⁵ cfu/ml angezüchtet. Es werden bis zu Zweidrittel des Propagats in den Abfülltank 8 geleitet, wo sich die vereinten Fermentauszüge befinden.

Durch Zugabe von Substrat wird dem verbliebenen Propagat Nährstoff zugeführt, um eine erneute Anreicherung der Mikroorganismenkultur auf die Zielkonzentration zu erreichen. Als Substrat können den Mikroorganismen im Propagator die Rohmaterialien für das Naturprodukt in zerkleinerter und geklärter Form zugeführt werden. Alternativ kann den Mikroorganismen im Propagator eine Nährlösung als Substrat hinzugefügt werden. Für diesen Fall wird ein weiterer Behälter vorgesehen, in dem die

Nährlösung vorhanden ist, die dann in den Propagator gesteuert geleitet wird. Dem Propagator wird ebenfalls Sauerstoff zugeführt, um aerobe Bedingungen im Propagator zu schaffen. Das kann beispielsweise durch Einleiten von Luft durch eine Belüftungsvorrichtung erfolgen.

Als Rohmaterialien werden erfindungsgemäß Früchte, Gemüse, Hülsenfrüchte, Pilze, Nüsse, Getreide, Reis, Kräuter, Wurzeln, Blätter, Blüten, einzeln oder in Kombination verwendet. Die Mischung an sich wird als nicht kritisch angesehen. Es ist praktisch jede Variation aus Früchten, Gemüsen, Hülsenfrüchten, Pilzen, Nüssen, Getreiden, Reis, Kräutern, Wurzeln, Blättern, Blüten, möglich. Eine bevorzugte Mischung für das erfindungsgemäße Verfahren ist eine Mischung aus Früchten, Gemüsen, Hülsenfrüchten, Nüssen und Kräutern.

Die Rohmaterialien können beispielsweise in einer Menge von 300 kg, bezogen auf einen 1000-Liter-Ansatz, verwendet werden. Vor der Fermentierung werden sie auf übliche Weise zerkleinert, beispielsweise durch Schneiden oder Schreddern. Die eingesetzten Rohmaterialien stammen bevorzugt aus biologischem Anbau.

Je nach Art des herzustellenden Naturprodukts werden in den Fermentierungsschritten a) und c) sowie bei der Propagat-Zugabe die gleichen oder unterschiedliche Mikroorganismen verwendet. In einer bevorzugten Ausführungsform werden Mikroorganismen, die aus Bakterien, Pilzen, Hefen und/oder Mischungen daraus gewählt sind, verwendet. Besonders bevorzugt werden Bakterien verwendet. Es kann im Prinzip jedes Bakterium für die Fermentation bzw. Propagatzugabe verwendet werden, solange es den Lebensmittelvorschriften entspricht. In einer bevorzugten

Ausführungsform des erfindungsgemäßen Verfahrens werden Bakterien verwendet, die aus der Gattung Lactobacillus gewählt sind. Beispiele dafür sind Lactobacillus casei und Lactobacillus rhamnosus. In einer weiteren Ausführungsform können die Lactobacillen im Gemisch mit anderen Bakterien, d. h. solche Bakterien, die nicht Lactobacillen sind, verwendet werden. Es werden bevorzugt probiotische Bakterien eingesetzt.

In der Regel wird eine Nährlösung, z.B. eine Lactoselösung, als Substrat für die Mikroorganismen vorgelegt.

Das erfindungsgemäße Verfahren umfasst mindestens zwei Prozessvarianten A und B, die nachfolgend im Einzelnen beschrieben werden.

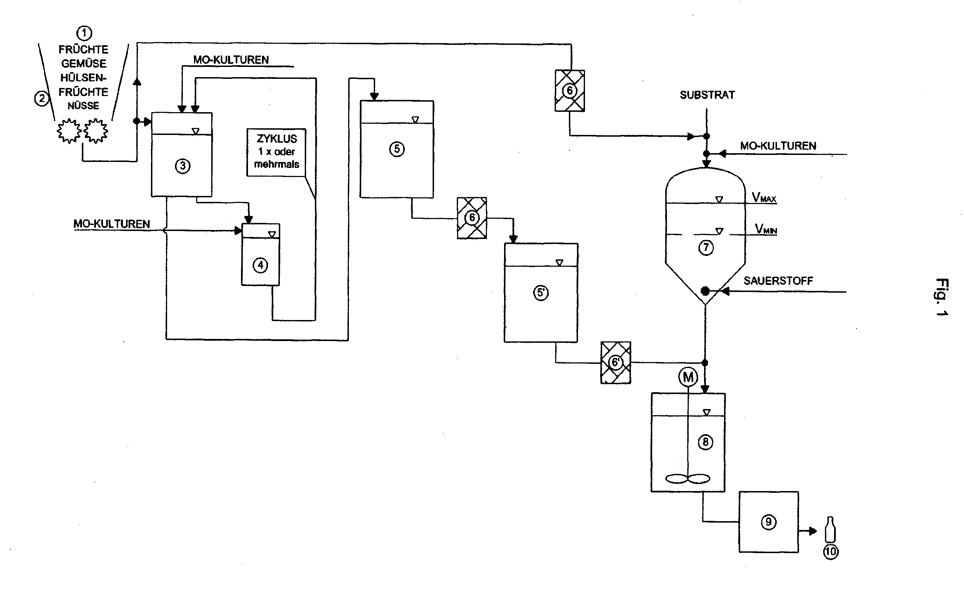

Die Prozessvariante A ist in Figur 1 näher erläutert. Figur 1 zeigt ein Prozessschema für eine Ausführungsform der Variante A des erfindungsgemäßen Verfahrens. Bei dieser Prozessvariante stammen alle Teilfermentauszüge aus dem ersten Fermentauszug, die dann mit dem ersten Fermentauszug vereint werden.

Die zerkleinerten Rohmaterialien, wie Früchte, Gemüse, Hülsenfrüchte und Nüsse, befinden sich in einem Behälter für Rohmaterialien 2, aus dem die Rohmaterialien in einen Hauptfermenter 3 geleitet werden. In den Hauptfermenter werden dann die für die Fermentation benötigten Mikroorganismenkulturen gegeben. Die Fermentation wird in der Regel bei einer Temperatur im Bereich von 20°C bis 35°C, bevorzugt 25°C bis 32°C, durchgeführt. Die Temperatur hängt davon ab, welcher Mikroorganismus oder welche Mikroorganismenmischung verwendet wird. In der Regel beträgt die Temperatur 28°C, wobei normalerweise in einem Zeitraum von 10 bis 20 Tagen fermentiert wird, was jeweils vom Ansatz und verwendeten Mikroorganismen abhängt.

Von diesem Ansatz wird dann mindestens ein Teil, beispielsweise bis zu einem Drittel, entnommen und in mindestens einen weiteren Fermenter, den Nebenfermenter 4, überführt. In diesem Nebenfermenter wird wieder unter Zuführung von Mikroorganismen bei einer Temperatur von 20°C bis 35°C fermentiert. Auch hier hängt die Temperatur wieder davon ab, welcher Mikroorganismus verwendet wird. Normalerweise beträgt die Temperatur 28°C. Nach erfolgter Teilfermentation wird dieser Ansatz aus dem Nebenfermenter 4 wieder in den Hauptfermenter 3 überführt. Dieser Zyklus kann sich auch mehrmals wiederholen.

Die Variante A beruht somit auf dem Prinzip, dass alle Teilfermentauszüge aus dem ersten Fermentauszug stammen und die Teilfermentierungen mit dem ersten Fermentauszug vereint werden.

Nachdem alle Teilfermentierungen mit dem ersten Fermentauszug in einen Sammeltank 5 überführt worden sind, werden diese ggf. filtriert und in einem weiteren Sammeltank 5' aufgenommen. Es schließt sich in einer nachfolgenden Filtriereinheit im Filter 6' eine weitere Klärung durch Filtrierung an. Das geklärte Filtrat wird in einen Abfülltank 8 überführt, in den aus einem Propagator 7 ein Propagat aus frischen Mikroorganismen gegeben wird. Nach dem Vermischen durch beispielsweise Rühren im Abfülltank 8 werden die Mikroorganismen enthaltenden Fermentauszüge in einer Füllvorrichtung 9 auf übliche Weise in Behälter 10, beispielsweise Flaschen, abgefüllt.

Gegebenenfalls werden die Fermentationsauszüge mit den Mikroorganismen bei einer Temperatur von 65°C bis 75°C in einem Erhitzer (nicht gezeigt) behandelt, vorzugsweise in einem Zeitraum von 60 bis 360 Sekunden, vorzugsweise 120 bis 300 Sekunden. Die Kurzzeiterhitzung dient dazu, die Mikroorganismen im Endprodukt zu inaktivieren.

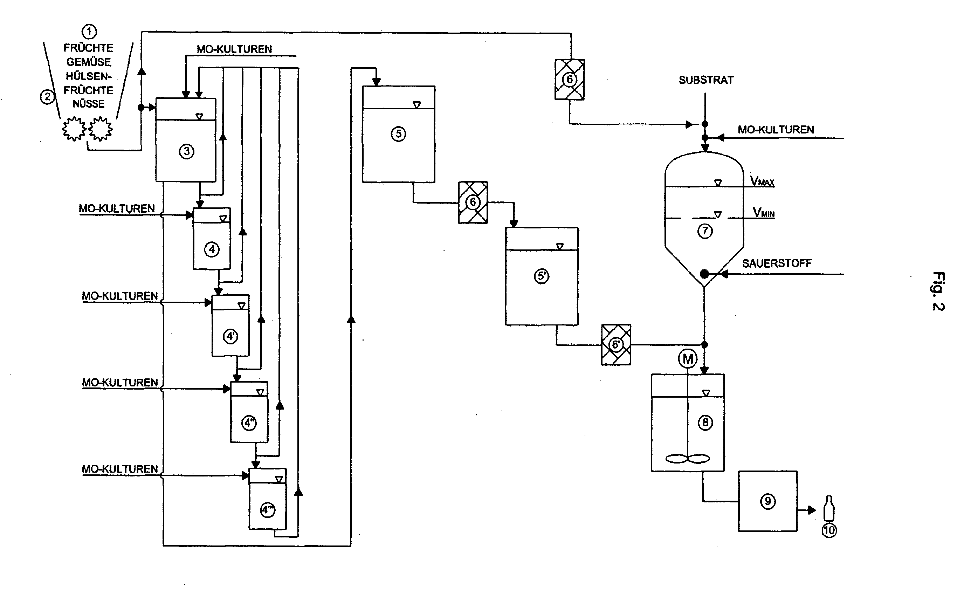

Die zweite Prozessvariante B ist in Figur 2 dargestellt. Bei dieser Prozessvariante stammt, wie bei Prozessvariante A, der erste Teilfermentauszug aus dem ersten Fermentauszug, wobei weitere Teilfermentierungen in nebeneinander geschalteten Nebenfermentern erfolgen und die einzelnen Teilfermentierungen mit dem noch verbliebenen ersten Fermentauszug vereint werden. Die zerkleinerten Rohmaterialien im Behälter 2 werden in den Hauptfermenter 3 überführt, in dem unter Zugabe von Mikroorganismen ein erster Fermentauszug hergestellt wird. Hinsichtlich der Prozessparameter wird auf die Ausführungen für Prozessvariante A verwiesen. Aus dem Hauptfermenter 3 wird dann ein Teil des ersten Fermentauszugs, beispielsweise bis zu einem Drittel, entnommen und in einen ersten Nebenfermenter 4' überführt, wobei die Fermentierung in Gegenwart von Mikroorganismen zur Bildung von mindestens einem Teilfermentauszug erfolgt. Nach der Fermentation wird dann aus dem Nebenfermenter 4' wieder ein Teil entnommen und in den Nebenfermenter 4" überführt, wo in Gegenwart von weiteren Mikroorganismen wieder eine Teilfermentation stattfindet. Diese Teilfermentationen lassen sich in beliebig vielen weiteren Nebenfermentern, wie in einem Nebenfermenter 4"', wiederholen.

Die einzelnen Teilfermentationen aus den Teilfermentern 4', 4" und 4'" werden wieder in den Hauptfermenter 3 überführt. Aus diesem erfolgt dann eine Überführung der vereinten Fermentauszüge in einen Sammeltank 5, wonach dann filtriert wird und ein Propagat aus Mikroorganismen hinzugefügt wird, wie dies bei der Prozessvariante A oben beschrieben wurde. Für den weiteren Verfahrensverlauf wird ebenfalls auf die Beschreibung der Prozessvariante A verwiesen.

Bei dieser Prozessvariante sind die Nebenfermenter miteinander verbunden, also in Reihe geschaltet. Diese Variante B unterscheidet sich von der Variante A dahingehend, dass auch größere Molekülstrukturen neben kleineren Molekülstrukturen erhalten bleiben.

Es können bei beiden Varianten beliebig viele Teilfermentationen durchgeführt werden. Es ist davon auszugehen, dass ein durch mehrere Teilfermentationen hergestelltes Naturprodukt eine besonders ungewöhnliche vielseitige Wirkungsbreite, insbesondere in der Immunmodulierung, aufweist.

Es ist weiterhin vorteilhaft, den Mikroorganismen enthaltenden vereinten Fermentauszügen vor der Abfüllung Additive hinzuzufügen. Alternativ können diese Additive auch vor dem Sammelbehälter 5' hinzugefügt werden.

Zu diesen Additiven zählen beispielsweise sekundäre Pflanzenstoffe, zum Beispiel Lutein, Vitamine, Mineralien, Fettsäuren, Polysaccharide, Polyglucane und/oder Extrakte aus Pilzen. Die Zusammenstellung dieser Additive erfolgt individuell und richtet sich nach dem gewünschten Wirkungsspektrum. Beispielsweise spielt bei der Auswahl der einzelnen Bestandteile der Konsumentenkreis eine Rolle. Während Frauen einen erhöhten Bedarf an Folsäure, Eisen, Magnesium, Zink und Biotin haben, bietet sich die Substitution von Vitamin K, B5, B9, B2 bei Männern an. Sportler haben einen erhöhten Bedarf an Natrium, Calcium, Magnesium, Zink, Vitamin D und C.

Das erfindungsgemäß hergestellte Produkt hat den Vorteil, dass es in niedriger Dosierung die Immunabwehr stärkt und ein besonders hohes antioxidatives Potenzial aufweist. Das Produkt ist für einen Zeitraum von mindestens drei Jahren im luftdicht verschlossenen Zustand haltbar.

Das erfindungsgemäß hergestellte Naturprodukt ist ein Bioprodukt und kann zur inneren und äußeren Verabreichung verwendet werden. Für die innere Verabreichung wird es in der Regel oral als Lebens- bzw. Nahrungsmittel oder Arzneimittel gegeben. Es kann in Form einer Flüssigkeit dargereicht werden. Es ist des Weiteren möglich, daraus ein Lyophilisat zu bilden, das bei Bedarf in Wasser oder einer anderen Flüssigkeit aufgelöst werden kann. Es ist ebenfalls vorteilhaft, das erfindungsgemäße Naturprodukt in Form von Tabletten, Pastillen, Granulaten, Ampullen, Tropfen oder Sprays zu verabreichen.

Das erfindungsgemäß hergestellte Naturprodukt kann ebenfalls auch äußerlich auf der Haut angewendet werden. Hier hat sich als günstig erwiesen, wenn das Naturprodukt, das beispielsweise in Form einer Flüssigkeit vorliegt, in Wickel oder Kompressen eingegeben wird und dann auf die betroffenen Hautstellen gelegt wird.

Man kann das erfindungsgemäß hergestellte Naturprodukt auch als Kosmetikprodukt verwenden, beispielsweise eingearbeitet in Cremes, Salben und Schäumen, womit es direkt auf die Haut aufgebracht wird. Die kosmetischen Produkte haben feuchtigkeitsspendende Eigenschaften und wirken als Anti-ageing Mittel.

Die hierin beschriebene Anlage ist für die Durchführung des erfindungsgemäßen Verfahrens geeignet. Mit dieser Anlage können beide Prozessvarianten durchgeführt werden.

Die beschriebene Anlage 1 weist mindestens zwei Fermenter 3; 4 und einen Propagator 7 zum Anzüchten von Mikroorganismen auf. Die einzelnen Vorrichtungen der beschriebenen Anlage lassen sich aus den Prozessschemen der Figuren 1 und 2 entnehmen.

In einer bevorzugten Ausführungsform der beschriebenen Anlage 1 bestehen die mindestens zwei Fermenter aus einem Hauptfermenter 3 und mindestens einem Nebenfermenter 4. In dem Hauptfermenter 3 wird der erste Fermentauszug hergestellt. In mindestens einem Nebenfermenter 4 wird mindestens ein weiterer Teilfermentauszug hergestellt. Für die Prozessvariante B sind die Nebenfermenter 4; 4'; 4"; 4"' in Reihe geschaltet und somit miteinander verbunden. Falls erforderlich, weist die Anlage ebenfalls nach dem Hauptfermenter und den Nebenfermentern Filter oder Sedimentationsvorrichtungen auf, um die Fermentauszüge zu klären.

Die beschriebene Anlage weist vorzugsweise weiterhin einen Sammeltank 5 auf, in den die vereinten Fermentauszüge geleitet werden. Dem Sammeltank 5 ist ein Filter 6 nachgeschaltet, der die Fermentauszüge reinigt, bzw. von unlöslichen Bestandteilen befreit. Die geklärten Fermentauszüge werden dann nochmals in einem Sammeltank 5' aufgefangen und dann wieder einer Filtervorrichtung β' zur weiteren Klärung zugeleitet.

Die beschriebene Anlage 1 weist einen Propagator 7 auf, in dem frische Mikroorganismenkulturen angezüchtet werden. Dieser Propagator 7 ist mit Zuleitungen verbunden, die Substrat und Mikroorganismenkultur in den Propagator leiten.

In einer Ausführungsform der beschriebenen Anlage wird nach dem Behälter für Rohmaterialien 2 ein Teilstrom als Substrat für das Propagat im Propagator 7 abgezweigt.

Die beschriebene Anlage weist weiterhin vorzugsweise einen Abfülltank 8 mit einer Rührvorrichtung auf, mit der die aus dem Propagator abgelassenen frischen Mikroorganismen und die geklärten vereinten Fermentauszüge homogen vermischt werden.

Die Anlage 1 kann einen Erhitzer (nicht gezeigt) aufweisen, in dem die Fermentauszüge mit den Mikroorganismen aus dem Propagator 7 auf eine Temperatur von 65° bis 75°C, vorzugsweise 68°C bis 72°C, erhitzt werden.

Eine Füllvorrichtung 9 dient dazu, das ggf. erhitzte Naturprodukt in geeignete Behälter, beispielsweise Flaschen 10 abzufüllen.

Die Flaschen werden dann sofort nach der Befüllung noch heiß luftdicht verschlossen.

## Patentansprüche

1. Verfahren zur Herstellung eines fermentierten Naturprodukts, mit den Schritten:
a) Fermentieren von Rohmaterialien für das Naturprodukt, die aus Früchten, Gemüsen, Hülsenfrüchten, Pilzen, Nüssen, Getreiden, Reis, Kräutern, Wurzeln, Blättern, Blüten, einzeln oder in Kombination gewählt sind, in Gegenwart von Mikroorganismen in einem Hauptfermenter (3) bei einer Temperatur von 20 bis 35°C zur Herstellung eines ersten Fermentauszugs;
b) Entnehmen mindestens eines Teils des ersten Fermentauszugs und Überführen dieses Teils in mindestens einen weiteren Nebenfermenter (4; 4'; 4"; 4"'), wobei der entnommene Teil bis zu einem Drittel des ersten Fermentauszugs bildet;
c) Fermentieren dieses Teils des Fermentauszugs in Gegenwart von Mikroorganismen bei einer Temperatur von 20 bis 35°C zur Bildung von mindestens einem Teilfermentauszug;
d) Überführen dieses mindestens einen Teilfermentauszugs in den Hauptfermenter (3) und Vermischen mit dem restlichen ersten Fermentauszug und
e) Zugeben eines Propagats aus Mikroorganismen in die vereinten
Fermentauszüge,
wobei in den Schritten a) und c) die Mikroorganismen in einer Menge von 10⁶ bis 10¹² Zellen/ml, bevorzugt 10⁸ bis 10¹⁰ Zellen/ml, hinzugefügt werden.

2. Verfahren nach Anspruch 1, **dadurch gekennzeichnet, dass** das Propagat aus Mikroorganismen in einer Menge in die vereinten Fermentauszüge hinzugefügt wird, die etwa zur Verdopplung der Mikroorganismenzahl führt.

3. Verfahren nach einem der Ansprüche 1 bis 2, **dadurch gekennzeichnet, dass** das Produkt in geeignete Behälter abgefüllt wird.

4. Verfahren nach mindestens einem der Ansprüche 1 bis 3, **dadurch gekennzeichnet, dass** alle Teilfermentauszüge aus dem ersten Fermentauszug stammen und die Teilfermentierungen mit dem ersten Fermentauszug vereint werden.

5. Verfahren nach mindestens einem der Ansprüche 1 bis 3, **dadurch gekennzeichnet, dass** der erste Teilfermentauszug aus dem ersten Fermentauszug stammt und weitere Teilfermentierungen in nebeneinander geschalteten Fermentern erfolgen und die einzelnen Teilfermentierungen mit dem ersten Fermentauszug vereint werden.

6. Verfahren nach mindestens einem der vorangegangenen Ansprüche, **dadurch gekennzeichnet, dass** das Propagat aus Mikroorganismen in einem Propagator angezüchtet wird.

7. Verfahren nach Anspruch 6, **dadurch gekennzeichnet, dass** den Mikroorganismen im Propagator die Rohmaterialien für das Naturprodukt als Substrat hinzugefügt wird.

8. Verfahren nach Anspruch 6, **dadurch gekennzeichnet, dass** den Mikroorganismen im Propagator eine Nährlösung als Substrat hinzugefügt wird.

9. Verfahren nach mindestens einem der vorangegangenen Ansprüche, **dadurch gekennzeichnet, dass** in den Fermentierungsschritten a) und c) sowie bei der Propagatzugabe die gleichen oder unterschiedliche Mikroorganismen verwendet werden.

10. Verfahren nach mindestens einem der vorangegangenen Ansprüche, **dadurch gekennzeichnet, dass** als Mikroorganismen Bakterien verwendet werden, die aus der Gattung Lactobacillus gewählt sind, wobei die Lactobacillen vorzugsweise im Gemisch mit anderen Bakterien verwendet werden.

11. Verfahren nach mindestens einem der vorangegangenen Ansprüche, **dadurch gekennzeichnet, dass** die vereinten Fermentauszüge mit dem Propagat bei einer Temperatur im Bereich von 65°C bis 75°C, vorzugsweise 68°C bis 72°C, vorzugsweise für einen kurzen Zeitraum von 60 bis 360 Sekunden und insbesondere 120 bis 300 Sekunden, hitzebehandelt werden.

12. Verfahren nach einem der vorangegangenen Ansprüche durchgeführt in einer Anlage (1) zur Herstellung eines fermentierten Naturprodukts, die
- mindestens zwei Fermenter (3; 4) und
- einen Propagator (7) und
- eine Füllvorrichtung (9)
aufweist.

13. Verfahren nach Anspruch 12, wobei die mindestens zwei Fermenter aus einem Hauptfermenter (3) und mehr als einem Nebenfermenter (4) bestehen, **dadurch gekennzeichnet, dass** die Nebenfermenter (4; 4'; 4", 4"') in Reihe geschaltet sind.

14. Verfahren nach mindestens einem der Ansprüche 12 und 13, **dadurch gekennzeichnet, dass** mit dem Propagator (7) Zuleitungen verbunden sind, die Substrat und Mikroorganismenkulturen in den Propagator leiten.

15. Verfahren nach mindestens einem der Ansprüche 12 bis 14, **dadurch gekennzeichnet, dass** sie weiterhin einen Erhitzer aufweist, in dem die vereinten Fermentauszüge mit den Mikroorganismen aus dem Propagator (7) auf eine Temperatur von 65°C bis 75°C, vorzugsweise 68°C bis 72°C, erhitzt werden.

## Claims

1. A method for the production of a fermented natural product, comprising the following steps:
a) fermenting raw materials for the natural product, selected individually or in combination from fruit, vegetables, legumes, mushrooms, nuts, grain, rice, herbs, roots, leaves, blossoms, in the presence of microorganisms in a main fermenter (3) at a temperature of 20 to 35°C for the production of a first fermentation extract;
b) removing at least part of the first fermentation extract, and transferring said part into at least one further secondary fermenter (4; 4'; 4"; 4"'), wherein the removed part forms up to a third of the first fermentation extract;
c) fermenting said part of the fermentation extract in the presence of microorganisms at a temperature of 20 to 35°C for forming of at least one partial fermentation extract;
d) transferring said at least one partial fermentation extract into the main fermenter (3) and mixing the same with the remaining first fermentation extract, and
e) adding a propagate of microorganisms to the combined fermentation extracts,
wherein in steps a) and c) the microorganisms are added in an amount of 10⁶ to 10¹² cells/ml, preferably 10⁸ to 10¹⁰ cells/ml.

2. The method according to claim 1, **characterized in that** the propagate comprised of microorganisms is added to the combined fermentation extracts at an amount leading to approximately the doubling of the number of microorganisms.

3. The method according to claims 1 to 2, **characterized in that** the product is filled into suitable containers.

4. The method according to at least one of the claims 1 to 3, **characterized in that** all partial fermentation extracts come from the first fermentation extract, and the partial fermentations are combined with the first fermentation extract.

5. The method according to at least one of the claims 1 to 3, **characterized in that** the first partial fermentation extract comes from the first fermentation extract, and further partial fermentations occur in successively connected fermenters, and the individual partial fermentations are combined with the first fermentation extract.

6. The method according to at least one of the previous claims, **characterized in that** the propagate is grown from microorganisms in a propagator.

7. The method according to claim 6, **characterized in that** the raw materials for the natural product are added to the microorganisms as a substrate.

8. The method according to claim 6, **characterized in that** a culture medium is added to the microorganisms in the propagator as a substrate.

9. The method according to at least one of the previous claims, **characterized in that** the same or different microorganisms are utilized in the fermentation steps a) and c), as well as with the propagate addition.

10. The method according to at least one of the previous claims, **characterized in that** bacteria are utilized as microorganisms, which are selected from the species of lactobacillus, wherein the lactobacilli are preferably utilized in a mixture with other bacteria.

11. The method according to at least one of the previous claims, **characterized in that** the combined fermentation extracts are heat-treated together with the propagate at a temperature in the range of 65°C to 75°C, preferably 68°C to 72°C, preferably for a short period of time from 60 to 360 seconds, and in particular from 120 to 300 seconds.

12. The method according to at least one of the previous claims, performed in a system (1) for the production of a fermented natural product, having
- at least two fermenters (3; 4), and
- one propagator (7), and
- one filling device (9).

13. The method according to claim 12, wherein the at least two fermenters consist of one main fermenter (3) and more than one secondary fermenter (4), **characterized in that** the secondary fermenters (4; 4'; 4"; 4"') are connected in series.

14. The method according to at least one of the claims 12 and 13, **characterized in that** feed lines are connected to the propagator (7), guiding the substrate and the microorganism cultures into the propagator.

15. The method according to at least one of the claims 12 to 14, **characterized in that** the same further comprises a heater, in which the combined fermentation extracts are heated together with the microorganisms from the propagator (7) to a temperature of 65°C to 75°C, preferably 68°C to 72°C.

## Revendications

1. Procédé pour la préparation d'un produit naturel fermenté, comportant les étapes :
a) fermentation de matières premières pour le produit naturel, qui sont choisies parmi les fruits, les légumes, les légumineuses, les champignons, les fruits à coques, les céréales, le riz, les herbes, les racines, les feuilles, les fleurs, à titre individuel ou en combinaison, en présence de microorganismes dans un fermenteur principal (3) à une température de 20 à 35°C, pour préparer un premier extrait fermenté ;
b) prélèvement d'au moins une partie du premier extrait fermenté, et transfert de cette partie dans au moins un fermenteur auxiliaire supplémentaire (4 ; 4' ; 4", 4"'), la partie prélevée formant jusqu'à un tiers du premier extrait fermenté ;
c) fermentation de cette partie de l'extrait fermenté en présence de microorganismes à une température de 20 à 35°C, pour former au moins un extrait fermenté partiel ;
d) transfert de ce ou ces extraits fermentés partiels dans le fermenteur principal (3) et mélange au reste du premier extrait fermenté, et
e) addition, aux extraits fermentés combinés, d'un produit de la propagation de
microorganismes,
les microorganismes étant dans les étapes a) et c) ajoutés en une quantité de 10⁶ à 10¹² cellules par millilitre, de préférence de 10⁸ à 10¹⁰ cellules par millilitre.

2. Procédé selon la revendication 1, **caractérisé en ce que** le produit de la propagation de microorganismes est ajouté aux extraits fermentés combinés en une quantité qui conduit approximativement au doublement du nombre des microorganismes.

3. Procédé selon l'une des revendications 1 à 2, **caractérisé en ce que** le produit est transvasé dans des récipients appropriés.

4. Procédé selon au moins l'une des revendications 1 à 3, **caractérisé en ce que** tous les extraits fermentés partiels proviennent du premier extrait fermenté, et les fermentations partielles sont combinées au premier extrait fermenté.

5. Procédé selon au moins l'une des revendications 1 à 3, **caractérisé en ce que** le premier extrait fermenté partiel provient du premier extrait fermenté, et que des fermentations partielles supplémentaires ont lieu dans des fermenteurs montés les uns à côté des autres, et les fermentations partielles individuelles étant combinées au premier extrait fermenté.

6. Procédé selon au moins l'une des revendications précédentes, **caractérisé en ce que** le produit de la propagation de microorganismes est cultivé dans un propagateur.

7. Procédé selon la revendication 6, **caractérisé en ce que** les matières premières pour le produit naturel sont ajoutées sous forme d'un substrat aux microorganismes dans le propagateur.

8. Procédé selon la revendication 6, **caractérisé en ce qu'**une solution nutritive est ajoutée en tant que substrat aux microorganismes dans le propagateur.

9. Procédé selon au moins l'une des revendications précédentes, **caractérisé en ce qu'**on utilise dans les étapes de fermentation a) et c) ainsi que lors de l'addition du produit de propagation les mêmes microorganismes, ou des microorganismes différents.

10. Procédé selon au moins l'une des revendications précédentes, **caractérisé en ce qu'**on utilise en tant que microorganismes des bactéries qui sont choisies parmi le genre *Lactobacillus,* les lactobacilles étant utilisés de préférence en mélange avec d'autres bactéries.

11. Procédé selon au moins l'une des revendications précédentes, **caractérisé en ce que** les extraits fermentés combinés sont, avec le produit de la propagation, soumis à un traitement thermique à une température comprise dans la plage de 65 à 75°C, de préférence de 68 à 72°C, de préférence pendant un bref laps de temps de 60 à 360 secondes et en particulier de 120 à 300 secondes.

12. Procédé selon l'une des revendications précédentes, mis en oeuvre dans une installation (1) de préparation d'un produit naturel fermenté, qui comprend
- au moins deux fermenteurs (3 ; 4) et
- un propagateur (7) et
- un dispositif de remplissage (9).

13. Procédé selon la revendication 12, dans lequel les au moins deux fermenteurs sont constitués d'un fermenteur principal (3) et de plus d'un fermenteur auxiliaire (4), **caractérisé en ce que** les fermenteurs auxiliaires (4 ; 4', 4", 4"') sont montés en série.

14. Procédé selon au moins l'une des revendications 12 et 13, **caractérisé en ce que** des conduites d'amenée, qui amènent le substrat et les cultures de microorganismes dans le propagateur, sont reliées au propagateur (7).

15. Procédé selon au moins l'une des revendications 12 à 14, **caractérisé en ce qu'**il comprend au moins un réchauffeur, dans lequel les extraits fermentés combinés sont, avec les microorganismes provenant du propagateur (7), chauffés à une température de 65 à 75°C, de préférence de 68 à 72°C.
